# EUROPÄISCHE PATENTSCHRIFT

(11) **EP 1 765 751 B1**
(45) Veröffentlichungstag und Bekanntmachung des Hinweises auf die Patenterteilung: **31.10.2007**
(21) Anmeldenummer: 05759413.7
(22) Anmeldetag: 07.07.2005
(51) Int. Cl.: C07C 29/145, C07C 29/149, C07C 31/20

(54) **VERFAHREN ZUR HERSTELLUNG VON 1,6-HEXANDIOL IN EINER REINHEIT VON ÜBER 99,5%**
METHOD FOR PRODUCTION OF 1,6-HEXANEDIOL WITH A PURITY IN EXCESS OF 99.5 %
PROCEDE DE PRODUCTION DE 1,6-HEXANEDIOL D'UNE PURETE SUPERIEURE A 99,5 %

(30) Priorität: 09.07.2004 DE 102004033557
(43) Veröffentlichungstag der Anmeldung: 28.03.2007
(73) Patentinhaber: BASF Aktiengesellschaft, 67056 Ludwigshafen (DE)
(72) Erfinder: HAUNERT, Andrea, 68163 Mannheim (DE); PINKOS, Rolf, 67098 Bad Dürkheim (DE); KRUG, Thomas, 67550 Worms (DE); SIRCH, Tilman, 67105 Schifferstadt (DE); KOCH, Michael, 68163 Mannheim (DE); TEBBEN, Gerd-Dieter, 37073 Göttingen (DE)
(86) Internationale Anmeldenummer: PCT/EP2005/007336
(87) Internationale Veröffentlichungsnummer: WO 2006/005504

(56) Entgegenhaltungen:
- WO-A-97/31882
- WO-A-20/04026798
- WO-A-20/04046072

## Beschreibung

Die Erfindung betrifft ein Verfahren zur Herstellung von 1,6-Hexandiol in einer Reinheit von >99,5 % aus Adipinsäuredialkylester, 6-Hydroxycapronsäurealkylester, 1,4-Cyciohexandion und 4-Hydroxycyclohexan-1-on enthaltenden Gemischen.

1,6-Hexandiol stellt einen gesuchten Monomerbaustein dar, der überwiegend auf dem Polyester- und Polyurethansektor eingesetzt wird. Bei diesen Anwendungen ist 1,4-Cyclohexandiol im 1,6-Hexandiol unerwünscht.

Geeignete Verfahren zur Herstellung von 1,6-Hexandiol sind in der DE-A 196 07 954 und DE-A 196 07 955 beschrieben. Hierbei wird Dicarbonsäurelbsung (DCS) zunächst mit einem C₁-C₁₀-Alkanol verestert und das erhaltene Veresterungsgemisch wird nach Entfernung überschüssigen Alkohols und anderer Leichtsieder durch Destillation aufgetrennt. Man erhält eine Esterfraktion, die im Wesentlichen frei ist von 1,4-Cyclohexandiolen.
Aus dieser Esterfraktion wird durch abschließende Hydrierung (Esterhydrierung) 1,6-Hexandiol mit einer Reinheit von mindestens 99 % hergestellt.

Obwohl das nach diesem Verfahren hergestellte C₆-Estergemisch weitgehend frei ist von 1,4-Cyclohexandiolen, findet man im Hydrieraustrag je nach Qualität der eingesetzten Dicarbonsäurelösung 1,4-Cyclohexandiole, die destillativ nicht vollständig abgetrennt werden können und damit im Rein-1,6-Hexandiol mit 0,05-0,5% auftauchen.

Edukte für die in DE-A 196 07 954 und DE-A 196 07 955 sowie WO 97/31882 beschriebenen Verfahren zur Herstellung von 1,6-Hexandiol sind die wässrigen Lösungen von Carbonsäuren, die bei der Oxidation von Cyclohexan zu Cyclohexanol und Cyclohexanon (vgl. Ullmann's Encyclopedia of Industrial Chemistry, 5. Ed., 1987, Vol. A8, S. 49) als Nebenprodukte entstehen, im Folgenden Dicarbonsäurelösung (DCL) genannt. Diese Dicarbonsäurelösungen enthalten (berechnet wasserfrei in Gew.-%) im Allgemeinen zwischen 10 und 40 % Adipinsäure, zwischen 10 und 40 % 6-Hydroxy-capronsäure, zwischen 1 und 10 % Glutarsäure, zwischen 1 und 10 % 5-Hydroxyvaleriansäure, zwischen 1 und 5 % 1,2-Cyclohexandiole, zwischen 1 und 5 % 1,4-Cyclohexandiole, zwischen2 und 10 % Ameisensäure sowie eine Vielzahl weiterer Mono- und Dicarbonsäuren, Ester, Oxo- und Oxa-Verbindungen, deren Einzelgehalte im Allgemeinen 5 % nicht übersteigen. Beispielsweise seien Essigsäure, Propionsäure, Buttersäure, Valeriansäure, Capronsäure, Oxalsäure, Malonsäure, Bernsteinsäure, 4-Hydroxybuttersäure und γ-Butyrolacton genannt.

Eine genauere Analyse der Dicarbonsäurelösung ergab als weitere Inhaltsstoffe 1,4-Cyclohexandion und 4-Hydroxycyclohexan-1-on in 0,01 bis 2 Gew.-%.
Beide Substanzen werden nach Veresterung und destillativer Aufreinigung der Ester nur unzureichend abgetrennt. In die abschließende Hydrierung des Estergemischs zu Hexandiol (Esterhydrierung) gelangendes 1,4-Cyclo-hexandion und 4-Hydroxycyclohexanon wird dort unter den Bedingungen der Esterhydrierung zu 1,4-Cyclohexandiol hydriert und führt zu einer nur unter hohen Ausbeuteverlusten des Hexandiol-Produktes abtrennbaren Verunreinigung.

Der vorliegenden Erfindung lag daher die Aufgabe zugrunde, Ein einfaches und kostengünstiges Verfahren zur Verfügung zu stellen, das aus 1,4-Cyclohexandion- und 4-Hydroxycyclohexan-1-on-haltiger Dicarbonsäurelösung 1,6-Hexandiol In reinerer Form liefert, ohne dass die Ausbeute an 1,6-Hexandiol sinkt.

Überraschenderweise wurde nun gefunden, dass diese Aufgabe gelöst wird, wenn das Estergemisch vor einer Destillation einer Hydrierung unterworfen wird.

Gegenstand der vorliegenden Erfindung ist ein Verfahren zur Herstellung von 1,6-Hexandiol mit einer Reinheit von >99,5 % durch Hydrierung von Adipinsäuredialkylester, 6-Hydroxycapronsäurealkylester und 1,4-Cyclohexandion und 4-Hydroxycyclohexan-1-on als Verunreinigungen enthaltenden Estergemischen, bei dem man
a) das erhaltene Veresterungsgemisch in einer ersten Destillationsstufe von überschüssigem Alkohol und Leichtsiedem befreit (im folgenden "Alkoholabtrennung),
b) aus dem Sumpfprodukt in einer zweiten Destillationsstufe eine Auftrennung in eine von 1,4-Cyclohexandiolen im wesentlichen freie Estertraktion und eine zumindest den größeren Teil der 1,4-Cyclohexandiole enthaltende Fraktion durchführt,
c) die im wesentlichen von 1,4-Cyclohexandiolen freie Esterfraktion katalytisch hydriert (im folgenden "Esterhydrierung") und
d) in einer Reindestillationsstufe aus dem Hydrieraustrag in an sich bekannter Weise Hexandiol-1,6 gewinnt,
dadurch gekennzeichnet, dass man vor Stufe a) und/oder vor Stufe b) das Estergemisch selektiv hydriert (im folgenden "Reinigungshydrierung"), bevorzugt jedoch vor Stufe b), der Destillation zur 1,4-Cyclohexandiolabtrennung.

Während der erfindungsgemäßen selektiven Reinigungshydrierung des in dem Estergemisch vorhandenen 1,4-Cyclohexandions und 4-Hydroxycyclohexan-1-ons werden die ebenfalls enthaltenen Adipinsäure und 6-Hydroxycapronsäurealkylester nicht zu Alkoholen hydriert. Dadurch wird vermieden, dass diese in der Reinigungshydrierung bevorzugt folgenden Destillation (Stufe b)) als Sumpfprodukt verloren gehen und die Hexandiolausbeute drastisch sinkt.

Überraschenderweise ist die gefundene Menge an 1,6-Hexandiol nicht abtrennbaren 1,4-Cyclohexandiolen nach der Hydrierung deutlich reduziert oder gar nicht mehr vorhanden, ohne dass die 1,8-Hexandiolausbeute gesunken ist.

Erfindungsgemäß zu verwendende Adipinsäuredialkylester sind insbesondere Ester der Adipinsäure mit niedermolekularen Alkoholen z.B. mit Alkoholen mit 1 bis 4 C-Atomen, oder diese enthaltende Estergemische kommen Edukte beliebiger Herkunft in Betracht, die aufgrund ihrer Herstellungsmethode 1,4-Cyclohexandion und 4-Hydroxycyclohexan-1-on als Verunreinigungen enthalten.

Bevorzugt verwendet man für das erfindungsgemäße Verfahren ein Estergemisch, wie es erhalten wird durch Veresterung eines Dicarbonsäuregemisches, das Adipinsäure, 6-Hydroxycapronsäure, 1,4-Cyclohexandiole, 1,4-Cyclohexandion und 4-Hydroxycyclohexan-1-on enthält und das als Nebenprodukt der Oxidation von Cyclohexan zu Cyclohexanon/Cyclohexanol mit Sauerstoff oder Sauerstoff enthaltenen Gasen durch Extraktion des Reaktionsgemisches mit Wasser anfällt, mit einem niedermolekularen Alkohol, bevorzugt n- oder i-Butanol, besonders bevorzugt Methanol (Esterfraktion a)).

Die Herstellung des Dicarbonsäuregemisches, der Esterfraktion (vorstehend als Stufen a) und b) bezeichnet) sowie das Verfahren zur Herstellung von 1,6-Hexandiol(Stufen c) und d)) ist bekannt und detailliert in DE-A 196 07 954 A und DE-A 196 07 955 beschrieben. Der gesamte Inhalt dieser Schriften wird daher durch Bezugnahme in die vorliegende Anmeldung aufgenommen.

Besonders bevorzugt wird ein Gemisch in der Reinigungshydrierung eingesetzt, das durch Entfernung des überschüssigen Veresterungsalkohols und/oder Leichtsiedem in einer ersten Destillationsstufe gewonnen wird (Alkoholabtrennung). Unter Leichtsiedem werden dabei Nebenprodukte verstanden, die niedriger sieden als die gewünschten Ester, insbesondere 1,2-Cyclohexandiole, Valerolacton, 5-Hydroxyvateriansäuremethylester, Glutarsäuredimethylester, Bernsteinsäuredimethylester. Die Alkoholabtrennung ist als Stufe 3 aus DE-A 196 07 955 und DE-A 196 07 954 bekannt, auf die hier ausdrücklich verwiesen wird.

Das nach der sogenannten Reinigungshydrierung erhaltene Estergemisch wird durch Auftrennung in einer weiteren Destillationsstufe (Stufe b) des erfindungsgemäßen Verfahrens), die an sich bekannt und z.B. in DE-A 196 07 954 als Stufe 4 beschrieben ist, in eine von 1,4-Cyclohexandiolen im Wesentlichen freie Esterfraktion und eine zumindest den größeren Teil der 1,4-Cyclohexandiole enthaltende Fraktion geteilt. Die von 1,4-Cyclohexandiolen im Wesentlichen freie Esterfraktion wird zum Hexandiol hydriert.

Die Reinigungshydrierung kann im erfindungsgemäßen Verfahren sowie in den DE-A 196 07 954 und DE-A 196 07 955 offenbarten Verfahren direkt nach der Veresterung d.h. vor der Alkoholabtrennung oder nach der Alkoholabtrennung, besonders bevorzugt nach der Alkoholabtrennung, durchgeführt werden.

Die Reinigungshydrierung erfolgt bei 20 bis 300°C, bevorzugt bei 50 bis 200°C, besonders bevorzugt bei 100 bis 160°C und bei einem Wasserstoffdruck von 1 bis 200 bar, bevorzugt bei 1 bis 100 bar H₂, besonders bevorzugt bei 10 bis 50 bar H₂ mit fest angeordneten, suspendierten Katalysator oder homogenen Katalysatoren.

Als Katalysatoren für die Reinigungshydrierung dienen in dem erfindungsgemäßen Verfahren bevorzugt heterogene Katalysatoren, die mindestens ein Metall der 8. bis 12. Gruppe des Periodensystems, beispielsweise Ruthenium, Osmium, Iridium, Platin, Palladium, Rhodium, Eisen, Kupfer, Kobalt, Nickel und Zink sowie Kombinationen dieser Metalle, enthalten. Diese Metalle können sowohl in Form der reinen Metalle als auch von deren Verbindungen, beispielsweise Oxyden oder Sulfiden, eingesetzt werden. Vorzugsweise werden Kupfer-, Nickel-, Kobalt-, Ruthenium- oder Palladiumkatalysatoren verwendet. Diese Katalysatoren können auf den üblichen Trägem, beispielsweise TiO₂, Al₂O₃, ZrO₂, SiO₂, Kohle oder deren Gemischen, aufgebracht sein. Die so erhaltenen, geträgerten Katalysatoren können in allen bekannten Konfektionierungsformen vorliegen. Beispiele sind Stränge oder Tabletten.

Die Verwendung geträgerter Palladium, Ruthenium und/oder Kupfer enthaltender Katalysatoren ist bevorzugt.

Für die Verwendung im Verfahren gemäß der vorliegenden Erfindung sind Raney-Kupfer, Raney-Nickel und Raney-Cobalt-Katalysatoren geeignet. Diese Raney-Katalysator können in allen bekannten Konfektionierungsformen, beispielsweise als Tabletten, Stränge oder Granulat vorliegen. Geeignete Raney-Kupfer Katalysatoren sind beispielsweise die Raney-Kupfer-Katalysatoren in Form von Nuggets, die aus der WO 99/03801 bekannt sind, auf die hier ausdrücklich Bezug genommen wird. Diese Katalysatoren weisen eine Korngröße der Nuggets von 2 bis 7 mm, ein Kupfergehalt von 40 bis 90 Gew.-%, eine Oberfläche nach Langmuir von-5 bis 50 m²/g, eine Kupferoberfläche von 0,5 bis 7m²/g, ein Hg-Porenvolumen von 0,01 bis 0,12 ml/g und einen mittleren Porendurchmesser von 50 bis 300 nm auf.

Besonders geeignet für die Verwendung in dem erfindungsgemäßen Verfahren ist weiterhin ein Katalysator, enthaltend auf Titandioxid-Formkörpern geträgertes Ruthenium, wobei die Titandioxid-Formkörper durch Behandeln von marktüblichem Titandioxid vor oder nach dem Formen mit 0,1 bis 30 Gew.-% einer Säure, in der Titandioxid schwer löslich ist, erhalten werden, der in dem erfindungsgemäßen Verfahren verwendet wird. Ruthenium kann dabei sowohl in Form des reinen Metalls als auch als dessen Verbindung, beispielsweise Oxyd oder Sulfid, eingesetzt werden.

Das katalytisch aktive Ruthenium wird nach an sich bekannten Verfahren, bevorzugt auf vorgefertigtes TiO₂ als Trägermaterial aufgebracht.

Ein für die Verwendung in dem Ruthenium enthaltenden Katalysator bevorzugt geeigneter Titandioxid-Träger kann entsprechend DE 197 38 464 durch Behandeln von marktüblichem Titandioxid vor oder nach dem Formen mit 0,1 bis 30 Gew.-% einer Säure, bezogen auf Titandioxid, in der das Titandioxid schwer löslich ist, erhalten werden. Bevorzugt wird Titandioxid in der Anatas-Modifikation verwendet. Als derartige Säure sind beispielsweise Ameisensäure, Phosphorsäure, Salpetersäure, Essigsäure oder Stearinsäure geeignet.

Die Aktivkomponente Ruthenium kann in Form einer Rutheniumsalzlösung auf den so erhaltenen Titandioxidträger in einer oder mehreren Tränkstufen aufgebracht werden. Anschließend wird der getränkte Träger getrocknet und gegebenenfalls calciniert. Es ist jedoch auch möglich Ruthenium aus einer Rutheniumsalzlösung, bevorzugt mit Natriumcarbonat, auf einen als Pulver in wässriger Suspension vorliegendes Titandioxids zu fällen. Die ausgefällten Niederschläge werden gewaschen, getrocknet, gegebenenfalls calciniert und verformt. Weiterhin können flüchtige Rutheniumverbindungen, wie beispielsweise Rutheniumacetylacetonat oder Rutheniumcarbonyl, in die Gasphase überführt werden und in an sich bekannter Weise auf den Träger aufgebracht werden (Chemical vapor deposition).

Die so erhaltenen, geträgerten Katalysatoren können in allen bekannten Konfektionierungsformen vorliegen. Beispiele sind Stränge, Tabletten oder Granulate. Vor ihrer Verwendung werden die Rutheniumkatalysatorvorläufer durch Behandlung mit Wasserstoffhaltigem Gas, bevorzugt bei Temperaturen über 100°C reduziert. Bevorzugt werden die Katalysatoren vor ihrem Einsatz im erfindungsgemäßen Verfahren bei Temperaturen von 0 bis 50°C, bevorzugt bei Raumtemperatur, mit sauerstoffhaltigen Gasgemischen, bevorzugt mit Luft-Stickstoffgemischen, passiviert. Es ist auch möglich, den Katalysator in oxidischer Form in den Hydrierreaktor einzubauen und unter Reaktionsbedingungen zu reduzieren.

Der erfindungsgemäß besonders bevorzugte Katalysator weist einen Rutheniumgehalt von 0,1 bis 10 Gew.-%, bevorzugt von 2 bis 6, bezogen auf das Gesamtgewicht des Katalysators aus katalytisch aktivem Metall und Träger, auf. Der erfindungsgemäße Katalysator kann einen Schwefelgehalt von 0,01 bis 1 Gew.-%, bezogen auf das Gesamtgewicht des Katalysators, aufweisen (Schwefel-Bestimmung: coulometrisch).

Die Rutheniumoberfläche beträgt dabei von 1 bis 20 m²/g, bevorzugt von 5 bis 15 und die BET-Oberfläche (bestimmt nach DIN 66 131) von 5 bis 500 m²/g, bevorzugt von 50 bis 200 m²/g.

Die erfindungsgemäßen Katalysatoren weisen ein Porenvolumen von 0,1 bis 1 ml/g auf. Weiterhin zeichnen sich die Katalysatoren durch einen Schneidhärte von 1 bis 100 N aus.

Falls die Aktivität und/oder Selektivität des Katalysators im Laufe der Betriebstätigkeit sinken sollte, kann der erfindungsgemäß verwendete Katalysator durch dem Fachmann bekannte Maßnahmen regeneriert werden. Hierzu zählt vorzugsweise eine reduktive Behandlung des Katalysators im Wasserstoffstrom bei erhöhter Temperatur. Gegebenenfalls kann der reduktiven Behandlung eine oxidative vorausgehen. Hierbei wird die Katalysatorschüttung mit einem molekularen Sauerstoff enthaltenem Gasgemisch, beispielsweise Luft, bei erhöhter Temperatur durchströmt. Weiterhin besteht die Möglichkeit, den Katalysator mit einem geeignetem Lösungsmittel, beispielsweise Ethanol oder THF, zu waschen und anschließend in einem Gasstrom zu trocknen.

Außerdem kann die Hydrierung durch literaturbekannte Hydrierreagenzien wie NaBH₄, LiAIH₄, etc. erfolgen bei 20 bis 200°C, bevorzugt bei 50 bis 200°C, besonders bevorzugt bei 100 bis 160°C erfolgen. Die Hydrierung kann kontinuierlich und diskontinuierlich, bevorzugt kontinuierlich durchgeführt werden.

Die anschließende Aufarbeitung des nach der Reinigungshydrierung erhaltenen-Estergemisches erfolgt wie in DE-A 196 07 954 A und DE-A 196 07 955 für den Esterstrom, der keiner Reinigungshydrierung unterworfen wurde, beschrieben.

Die Hydrierung der Ester (Esterhydrierung) zu Hexandiol erfolgt in an sich bekannter Weise wie in der DE-A 196 07 954 beschrieben, insbesondere bei 20 bis 300°C und bei einem Druck von 1 bis 50 bar (bei Hydrierungen in der Gasphase über einem fest angeordneten Katalysator) oder bei einem Druck im Bereich von 30 bis 350 bar (bei Hydrierungen in der Flüssigphase mit fest angeordnetem oder suspendiertem Katalysator). Die Hydrierung kann diskontinuierlich, bevorzugt kontinuierlich erfolgen.

Der Hydrieraustrag der Esterhydrierung besteht im Wesentlichen aus 1,6-Hexandiol und dem Veresterungsalkohol. Weitere Bestandteile sind 1,5-Pentandiol, 1,4-Butandiol, 1,2-Cyclohexandiole sowie kleine Mengen an Monoalkoholen mit 1 bis 6 C-Atomen und Wasser.

Der Hydrieraustrag der Esterhydrierung wird in der nächsten Stufe in z.B. ein Membransystem oder bevorzugt eine Destillationskolonne eingespeist und in den Veresterungsalkohol, der zusätzlich den größten Teil der weiteren leichtsiedenden Komponenten enthält und einen Strom, der überwiegend 16-Hexandiol neben 1,5-Pentandiol, aufgetrennt Dabei werden bei einem Druck von 10 bis 1 500 mbar, bevorzugt 30 bis 1 200 mbar, besonders bevorzugt 50 bis 1 000 mbar Kopftemperaturen von 0 bis 120°C, bevorzugt 20 bis 100°C, besonders bevorzugt 30 bis 90°C sowie Sumpftemperaturen von 100 bis 270°C, bevorzugt 140 bis 260°C, besonders bevorzugt 160 bis 250°C eingestellt

Der 1,6-Hexandiol enthaltende Stoffstrom wird in einer Kolonne gereinigt. Dabei werden 1,5-Pentandiol, die 1,2-Cyclohexandiole sowie weitere eventuell vorhandene Leichtsieder über Kopf abgetrennt. Sollen die 1,2-Cyclohexandiole und/oder 1,5-Pentandiol als zusätzliche Wertprodukte gewonnen werden, so können diese in einer weiteren Kolonne aufgetrennt werden. Über den Sumpf werden eventuell vorhandene Hochsieder ausgeschleust.

1,6-Hexandiol wird mit deutlich reduziertem 1,4-Cyclohexandiolgehalt von 0,005 bis 0,1 Gew.-% mit einer Reinheit > 99,5 % aus einem Seitenstrom der Kolonne entnommen.

Dabei werden bei Drücken von 1 bis 1 000 bar, bevorzugt 5 bis 800 mbar, besonders bevorzugt 20 bis 500 mbar Kopftemperaturen von 50 bis 200°C, bevorzugt 60 bis 150°C und Sumpftemperaturen von 130 bis 270°C, bevorzugt 150 bis 250°C eingestellt.

Sollen nur kleinere Mengen 1,6-Hexandiol hergestellt werden, so können die Stufen auch in einer diskontinuierlichen fraktionierten Destillation zusammengefasst werden.

Das erfindungsgemäße Verfahren stellt somit eine einfache und kostengünstige Vorgehensweise zur Gewinnung von hochreinem 1,6-Hexandiol mit minimalen Mengen 1,4-Cyclohexandiolen dar.

Die nachfolgenden Beispiele erläutern die Erfindung ohne sie zu begrenzen.

### Beispiel 1: Reinigungshydrierung diskontinuierlich

### Beispiel 1a:

0,15 kg Estergemisch (ca. 90 Gew.-% Adipinsäuredimethylester und 10 Gew.-% Hydroxycapronsäuremethylester), das etwa 1500 ppm 1,4-Cyclohexandion enthielt, wurden mit 10 g eines Pd/Al₂O₃ Katalysators bei 130 °C und 30 bar Wasserstoff 180 min. umgesetzt. Der Austrag enthielt ca. 10 Gew.-% Hydroxycapronsäuremethylester, ca. 90% Adipinsäuredimethylester, 380 ppm 1,4-Cyclohexandiol, 200 ppm 1,4-Cyclohexandion und 1050 ppm 4-Hydroxycyclohexanon.

### Beispiel 1b:

0,15 kg Estergemisch (ca. 90 Gew.-% Adipinsäuredimethylester und 10-Gew.-% Hydroxycapronsäuremethylester), das etwa 1500 ppm 1,4-Cyclohexandion enthielt, wurden mit 10 g eines 2 % Ru/Al₂O₃ Katalysators bei 130 °C und 30 bar Wasserstoff 180 min umgesetzt. Der Austrag enthielt ca. 10 Gew.-% Hydroxycapronsäuremethylester, ca. 90 Gew.-% Adipinsäuredimethylester, 1490 ppm 1,4-Cyclohexandiol, 0 ppm 1,4-Cyclohexandion und 0 ppm 4-Hydroxycyclohexanon.

### Beispiel 1c:

0,15 kg Estergemisch (ca. 90 Gew.-% Adipinsäuredimethylester und 10 Gew.-% Hydroxycapronsäuremethylester), das etwa 1500 ppm 1,4-Cyclohexandion enthielt, wurden mit 10 g eines 5% Ru/SiO₂ Katalysators bei 130°C und 30 bar Wasserstoff 150min umgesetzt. Der Austrag enthielt ca. 10 Gew.-% Hydroxycapronsäuremethylester, ca. 90 Gew.-% Adipinsäuredimethylester, 1720 ppm 1,4-Cyclohexandiol, 0 ppm 1,4-Cyclohexandion und 0 ppm 4-Hydroxycyclohexanon.

### Beispiel 1d:

0,15 kg Estergemisch (ca. 90 Gew.-% Adipinsäuredimethylester und 10 Gew.-% Hydroxycapronsäuremethylester), das etwa 1500 ppm 1,4-Cyclohexandion enthielt, wurden mit 10 g eines 5 % Ru/TiO₂ Katalysators bei 130°C und 30 bar Wasserstoff 150min umgesetzt. Der Austrag enthielt ca. 10 Gew.-% Hydroxycapronsäuremethylester, ca. 90 Gew.-% Adipinsäuredimethylester, 1560 ppm 1,4-Cyclohexandiol, 0 ppm 1,4-Cyclohexandion und 0 ppm 4-Hydroxycyclohexanon.

### Beispiel 1e:

0,15 kg Estergemisch (ca. 90 Gew.-% Adipinsäuredimethylester und 10 Gew.-% Hydroxycapronsäuremethylester), das etwa 1500 ppm 1,4-Cyclohexandion enthielt, wurden mit 10 g eines 2 % Ru/C Katalysators bei 130 °C und 30 bar Wasserstoff 150min umgesetzt. Der Austrag enthielt ca. 10 Gew.-% Hydroxycapronsäuremethylester, ca. 90 Gew.-% Adipinsäuredimethylester, 1800 ppm 1;4-Cyclohexandiol, 0 ppm 1,4-Cyclohexandion und 0 ppm 4-Hydroxycyclohexanon.

### Beispiel 1f:

0,15 kg Estergemisch (ca. 90 Gew.-% Adipinsäuredimethylester und 10 Gew.-% Hydroxycapronsäuremethylester), das etwa 1500 ppm 1,4-Cyclohexandion enthielt, wurden mit 10 g eines 1 % Ru/αAl₂O₃ Katalysators bei 130°C und 30 bar Wasserstoff 180 min umgesetzt. Der Austrag enthielt ca. 10 Gew.-% Hydroxycapronsäuremethylester, ca. 90 Gew.-% Adipinsäuredimethylester, 2400ppm 1,4-Cyclohexandiol, 0 ppm 1,4-Cyclohexandiol und 0 ppm 4-Hydroxycyclohexanon.

### Beispiel 1e:

0,15 kg Estergemisch hergestellt nach DE-A 196 07 954 (nach Stufe a); ca. 5 Gew.-% Adipinsäuredimethylester und 16 Gew.-% Hydroxycapronsäuremethylester), das etwa 0,07 Gew.-% 1,4-Cyclohexandion und 0,4 Gew.-% 4-Hydroxycyclohexanon enthielt wurde mit 10 g eines 5 % Ru/TiO₂ Katalysators bei 150°C und 30 bar Wasserstoff 150 min umgesetzt. Der Austrag enthielt ca. 16 Gew.-% Hydroxycapronsäuremethylester, ca. 25 % Adipinsäuredimethylester, 0 ppm 1,4-Cyclohexandion und 0 ppm 4-Hydroxycyclohexanon.

### Beispiel 2: kontinuierliche Hydrierung, vor Stufe b)

Ein C₆-Estergemisch hergestellt nach DE-A 196 07 954 (nach Alkohol und Leichtsiederabtrennung vor Stufe b)); ca. 25 Gew.-% Adipinsäuredimethylester und 16 Gew.-% Hydroxycapronsäuremethylester), das etwa 0,07 Gew.-% 1,4-Cyclohexandion und 0,4 Gew.-% 4-Hydroxycyclohexanon enthielt, wurde kontinuierlich in einem Festbettreaktor an einem 2 % Ru/TiO₂ Katalysator, der zuvor im Wasserstoffstrom bei 180°C aktiviert wurde, umgesetzt. Hydrierbedingungen: Rieselbett, 250 ml Katalysator, 1,5 mm Stänglinge, Zulauf 750-1500 g/h, kein Umlauf, 30 bar, 150°C). Der Austrag enthielt ca. 16 % Hydroxycapronsäuremethylester, ca. 25 Gew.-% Adipinsäuredimethylester, <20 ppm 1,4-Cyclohexandion und 0 ppm 4-Hydroxycyclohexanon.

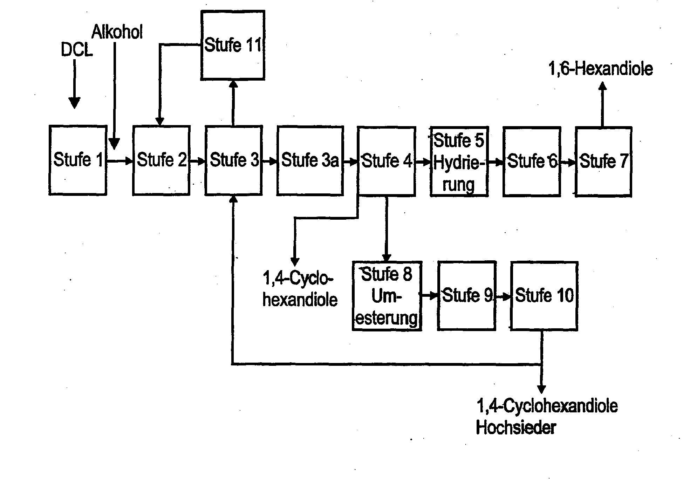

### Beispiele 3: Herstellung von 1,6-Hexandiol (siehe Zeichnung 1)

### Stufe 1: (Entwässerung)

0,1 kg Dicarbonsäurelösung (Adipinsäure ca.17 Gew.-%, ca. 13 % 6-Hydroxycapronsäure, ca. 1,5 Gew.-% 1,4-Cyclohexandiole, ca. 0,08 Gew.-% 1,4 Cyclohexandion, ca. 45 % Wasser) wurden kontinuierlich in einer Destillationsapparatur (dreibödige Glockenbodenkolonne mit außenliegendem Öl-Heizkreislauf, Öltemperatur 150°C, Bodenvolumen je ca. 25 ml, Zulauf über den Glockenböden) mit aufgesetzter Füllkörperkolonne (ca. 4 theoretische Trennstufen, kein Rücklauf am Kopf) destilliert. Als Kopfprodukt wurden 0,045 kg erhalten mit einem Ameisensäuregehalt im Wasser von ca. 3 %. Im Sumpfstrom (5,5 kg) betrug der Wassergehalt ca. 0,4 %.

### Stufe 2: (Veresterung)

5,5 kg des Sumpfstroms aus Stufe 1 wurde mit 8,3 kg Methanol und 14 g Schwefelsäure umgesetzt. Die Säurezahl des Austrags abzüglich Schwefelsäure betrug ca.10 mg KOH/g.

### Stufe 3: (Alkoholabtrennung)

In einer Kolonne wurden der Veresterungsstrom aus Stufe 2 destilliert (1015 mbar, 65°C Kopftemperatur, bis 125°C Sumpftemperatur). Über Kopf wurden 7,0 kg abgezogen. Als Sumpfprodukt wurden 6,8 kg erhalten.

### Stufe 3a:

Das Sumpfprodukt aus Stufe 3 wurde kontinuierlich in einem Festbettreaktor an einem 2 % Ru/TiO2 Katalysator, der zuvor im Wasserstoffstrom bei 180°C aktiviert wurde, umgesetzt. (Hydrierbedingungen: Rieselbett, 250 ml Katalysator, 1,5 mm Stänglinge, Zulauf 750 g/h, kein Umlauf, 30 bar, 150°C). Der Austrag enthielt ca. 16 % Hydroxycapronsäuremethylester, ca. 25 % Adipinsäuredimethylester, <20 ppm 1,4 -Cyclohexandion und 0 ppm 4-Hydroxycyclohexanon.

### Stufe 4: (1,4-Cyclohexandiolabtrennung)

In einer 50 cm Füllkörperkolonne wurde der Strom aus Stufe 3a fraktioniert destilliert (1 mbar, 70 bis 90°C Kopftemperatur, bis 180°C Sumpftemperatur). Im Sumpf fanden sich die 1,4-Cyclohexandiole.

Als Leichsieder wurden 0,6 kg abdestilliert-(1,2-Cyclohexandiole, Valerolacton, 5-Hydroxy-valeriansäuremethylester, Glutarsäuredimethylester, Bernsteinsäuredimethylester u.a.); als überwiegend Adipinsäuredimethylester und 6-Hydroxycapronsäuremethylester enthaltende fraktion wurden 4,3 kg erhalten.

### Stufe 5: (kontinuierliche Hydrierung, Teilstrom)

2,7 kg C₆-Estergemisch aus Stufe 4 wurden kontinuierlich in einem 25 ml Reaktor an einem Katalysator hydriert (Katalysator, 60 % CuO, 30 % Al₂O₃, 10 % Mn₂O₃), der zuvor im Wasserstoffstrom bei 180°C aktiviert worden ist. Hydrierbedingungen: Zulauf 20 g/h, kein Umlauf, 220 bar, 220°C). Der Ester-Umsatz betrug 99,5 %, die 1,6-Hexandiolselektivität betrug über 99 %. Im Hydrieraustrag befinden sich ca. 150 bis 250 ppm 1,4-Cyclohexandiole.

### Stufe 6 und 7: (Hexandiolreinigung)

2,5 kg des Hydrieraustrags aus Stufe 5 wurden fraktioniert destilliert (Destillationsblase mit aufgesetzter 70 cm füllkörperkolonne, Rücklaufverhältnis 2). Bei 1013 mbar wurden 0,5 kg Methanol abdestilliert und nach Anlegen von Vakuum (20 mbar Stufe 7) destillierten überwiegend die 1,2-Cyclohexandiole und 1,5-Pentandiol ab. Danach (Sdp. 146°C) destillierte 1,6-Hexandiol mit einer Reinheit von >99,7 % ab. Hauptnebenprodukt sind ca. 200-300 ppm 1,4-Cyclohexandiol.

### Stufe 8:

2,9 kg des Sumpfaustrages der Stufe 4 wurden mit 3,8 kg Methanol und 3,8 g Tetra-i-propyl-titanat versetzt und kontinuierlich in einem 1 m langen, 440 ml fassenden Rohrreaktor, der mit 3 mm V2A-Ringen gefüllt war, umgesetzt. Die mittlere Verweilzeit betrug ca. 2 h.

### Stufe 9:

Der Austrag aus Stufe 8 wurde analog der in Stufe 3 beschriebenen Apparatur fraktioniert destilliert. Bei 65°C Kopftemperatur wurden 3,5 kg abdestilliert (überwiegend Methanol). Im Sumpf verblieben 2,2 kg.

### Stufe 10:

Der Sumpf aus Stufe 9 wurde analog Stufe 4 bis zu einer Sumpftemperatur von 160°C fraktioniert destilliert. Als Destillat wurden 1,3 kg erhalten, das direkt hydriert oder in die 4. Stufe zurückgeführt werden kann. (Zusammensetzung: 52 Gew.-% 6-Hydroxycapronsäuremethylester, 31 Gew.-% Adipinsäuredimethylester, 5 Gew.-% Glutarsäuredimethylester, 4 Gew.-% 5-Hydroxycapronsäuremethylester sowie eine Vielzahl weiterer, mengenmäßig unbedeutender Komponenten).

### Stufe 11:

7 kg des Kopfproduktes der Stufe 3 wurden an einer 20 cm Füllkörperkolonne bei 1015 mbar fraktioniert destilliert. Es wurden 0,8 kg Vorlauffraktion bei 59 bis 65°C Kopftemperatur erhalten, die neben vorwiegend Methanol, C₁- C₄-Monoethylester enthielt. Bei 65°C Kopftemperatur wurden 5,6 kg Methanol mit einer Reinheit > 99,5 % erhalten. Der Sumpf (0,6 kg) bestand überwiegend aus Wasser.

## Patentansprüche

1. Verfahren zur Herstellung von 1,6-Hexandiol durch Hydrierung von Adipinsäuredialkylester, 6-Hydroxycapronsäureaikylester und 1,4-Cyclohexandion und 4-Hydroxy-cyclohexan-1-on als Verunreinigungen enthaltenden Estergemischen, bei dem man
a) das erhaltene Veresterungsgemisch in einer ersten Destillationsstufe von überschüssigem Alkohol und Leichtsiedem befreit,
b) aus dem Sumpfprodukt in einer zweiten Destillationsstufe eine Auftrennung in eine von 1,4-Cyclohexandiolen im wesentlichen freie Esterfraktion und eine zumindest den größeren Teil der 1,4-Cyclohexandiole enthaltende Fraktion durchführt,
c) die im wesentlichen von 1,4-Cyclohexandiolen freie Esterfraktion katalytisch hydriert und
d) in einer Reindestillationsstufe aus dem Hydrieraustrag in an sich bekannter Weise Hexandiol-1,6 gewinnt,
**dadurch gekennzeichnet, dass** man vor Stufe a) und/oder vor Stufe b) das Estergemisch selektiv hydriert (Reinigungshydrierung).

2. Verfahren nach Anspruch 1, **dadurch gekennzeichnet, dass** die Reinigungshydrierung an einem heterogenen Katalysator durchgeführt wird.

3. Verfahren nach einem der Ansprüche 1 oder 2, **dadurch gekennzeichnet, dass** die Reinigungshydrierung bei 20 bis 300°C und 1 bis 200 bar H₂ durchgeführt wird.

4. Verfahren nach einem der Ansprüche 1 bis 3, **dadurch gekennzeichnet, dass** die Reinigungshydrierung an geträgerten Palladium, Ruthenium und/oder Kupfer enthaltenden Katalysatoren durchgeführt wird.

5. Verfahren nach einem der Ansprüche 1 bis 4, **dadurch gekennzeichnet, dass** die Reinigungshydrierung an auf Titandioxid-Formkörpern geträgertem Ruthenium durchgeführt wird.

6. Verfahren nach einem der Ansprüche 1 bis 5, **dadurch gekennzeichnet, dass** man als Edukt ein Estergemisch verwendet wie es erhalten wird,
a) durch Veresterung eines Dicarbonsäuregemisches, das Adipinsäure, 6-Hydroxy-capronsäure und geringe Mengen 1,4-Cyclohexandiole enthält und das als Nebenprodukt bei der Oxidation von Cyclohexan zu Cyclohexanon/Cyclohexanol mit Sauerstoff oder Sauerstoff enthaltenden Gasen und durch Wasserextraktion des Reaktionsgemisches anfällt, mit einem niedermolekularen Alkohol (Esterfraktion a)).

7. Verfahren nach einem der Ansprüche 1 bis 6, **dadurch gekennzeichnet, dass** man als Edukt ein Estergemisch verwendet, bei dem aus Esterfraktion a) überschüssiger Alkohol und Leichtsieder entfernt wurden (Esterfraktion b)).

## Claims

1. A process for preparing 1,6-hexanediol by hydrogenating dialkyl adipates, alkyl 6-hydroxycaproates and 1,4-cyclohexanedione and 4-hydroxycyclohexan-1-one as ester mixtures comprising impurities, by
a) freeing the resulting esterification mixture of excess alcohol and low boilers in a first distillation stage,
b) carrying out a separation of the bottom product in a second distillation stage into an ester fraction substantially free of 1,4-cyclohexanediols and a fraction comprising at least the majority of the 1,4-cyclohexanediols,
c) catalytically hydrogenating the ester fraction substantially free of 1,4-cyclohexanediols and
d) in a purifying distillation stage, obtaining 1,6-hexanediol from the hydrogenation effluent in a manner known per se,
which comprises selectively hydrogenating the ester mixture before stage a) and/or before stage b) (purifying hydrogenation).

2. The process according to claim 1, wherein the purifying hydrogenation is carried out over a heterogeneous catalyst.

3. The process according to either of claims 1 and 2, wherein the purifying hydrogenation is carried out at from 20 to 300°C and from 1 to 200 bar of H₂.

4. The process according to any of claims 1 to 3, wherein the purifying hydrogenation is carried out over supported palladium-, ruthenium- and/or copper-comprising catalysts.

5. The process according to any of claims 1 to 4, wherein the purifying hydrogenation is carried out over ruthenium supported on shaped titanium dioxide bodies.

6. The process according to any of claims 1 to 5, wherein the reactant used is an ester mixture as obtained
a) by esterifying with a low molecular weight alcohol a dicarboxylic acid mixture which comprises adipic acid, 6-hydroxycaproic acid and small amounts of 1,4-cyclohexanediols and which is obtained as a by-product in the oxidation of cyclohexane to cyclohexanone/cyclohexanol with oxygen or oxygen-comprising gases and by water extraction of the reaction mixture (ester fraction a)).

7. The process according to any of claims 1 to 6, wherein the reactant used is an ester mixture in which excess alcohol and low boilers have been removed from ester fraction a) (ester fraction b)).

## Revendications

1. Procédé de préparation de 1,6-hexanediol par hydrogénation de mélanges d'esters contenant un ester dialkylique d'acide adipique, un ester alkylique d'acide 6-hydroxycaproïque et de la 1,4-cyclohexanedione et de la 4-hydroxy-cyclohexan-1-one en tant qu'impuretés, dans lequel :
a) dans une première étape de distillation, on débarrasse le mélange d'estérification obtenu de l'alcool excédentaire et des composants à bas point d'ébullition,
b) dans une deuxième étape de distillation, on opère une séparation du résidu de distillation en une fraction ester sensiblement exempte de 1,4-cyclohexanediols et une fraction contenant au moins la majeure partie des 1,4-cyclohexanediols,
c) on opère une hydrogénation catalytique de la fraction ester sensiblement exempte de 1,4-cyclohexanediols, et
d) on récupère du 1,6-hexanediol du produit d'hydrogénation, dans une étape de distillation pure, de manière en soi connue,
**caractérisé en ce que**, avant l'étape a) et/ou avant l'étape b), on hydrogène sélectivement le mélange d'esters (hydrogénation de purification).

2. Procédé suivant la revendication 1, **caractérisé en ce que** l'hydrogénation de purification est entreprise sur un catalyseur hétérogène.

3. Procédé suivant l'une quelconque des revendications 1 ou 2, **caractérisé en ce que** l'hydrogénation de purification est entreprise à une température de 20 à 300°C et sous 1 à 200 bar de H₂.

4. Procédé suivant l'une quelconque des revendications 1 à 3, **caractérisé en ce que** l'hydrogénation de purification est entreprise sur des catalyseurs sur support contenant du palladium, du ruthénium et/ou du cuivre.

5. Procédé suivant l'une quelconque des revendications 1 à 4, **caractérisé en ce que** l'hydrogénation de purification est entreprise sur du ruthénium déposé sur des corps façonnés en dioxyde de titane.

6. Procédé suivant l'une quelconque des revendications 1 à 5, **caractérisé en ce que** l'on utilise en tant qu'éduit un mélange d'esters tel qu'obtenu :
a) par estérification d'un mélange d'acides dicarboxyliques, contenant de l'acide adipique, de l'acide 6-hydroxycaproïque et de petites quantités de 1,4-cyclohexanediols, et qui se forme en tant que sous-produit lors de l'oxydation du cyclohexane en cyclohexanone/cyclohexanol par de l'oxygène ou des gaz contenant de l'oxygène et par extraction à l'eau du mélange réactionnel, avec un alcool à bas poids moléculaire (fraction ester a)).

7. Procédé suivant l'une quelconque des revendications 1 à 6, **caractérisé en ce que** l'on utilise en tant qu'éduit un mélange d'esters dans lequel on a éliminé de la fraction ester a) l'alcool excédentaire et des composants à bas point d'ébullition (fraction ester b)).
